# NEW EUROPEAN PATENT SPECIFICATION

(11) **EP 2 249 785 B2**
(45) Date of publication and mention of the opposition decision: **25.09.2019**
(45) Mention of the grant of the patent: 14.12.2016
(21) Application number: 08744602.7
(22) Date of filing: 28.03.2008
(51) Int. Cl.: A61K 8/44, A61K 8/55, A61Q 11/00, A61K 31/198, A61K 8/46, A61K 8/81

(54) **ARGININE SALTS AND THEIR USES FOR THE TREATMENT OF ILLNESSES IN THE ORAL CAVITY**
ARGININSALZE UND DEREN ANWENDUNG ZUR BEHANDLUNG VON ERKRANKUNGEN DER MUNDHÖLE
SELS D ARGININE ET LEURS UTILISATIONS POUR LE TRAITEMENT DE MALADIES DE LA CAVITÉ BUCCALE

(30) Priority: 08.02.2008 US 27432; 08.02.2008 US 27420; 08.02.2008 US 27431; 09.02.2008 US 27442
(43) Date of publication of application: 17.11.2010
(60) Divisional application: 16172302.8
(73) Proprietor: Colgate-Palmolive Company, New York, NY 10022 (US)
(72) Inventor: PRENCIPE, Michael, Princeton Junction, NJ 08550 (US); XU, Guofeng, Plainsboro, NJ 08536 (US); SUBRAMANYAM, Ravi, Belle Mead, NJ 08502 (US); WU, Donghui, Bridgewater, NJ 08807 (US); CUMMINS, Diane, Livingston, NJ 07039 (US); SULLIVAN, Richard, J., Atlantic Highlands, NJ 07716 (US); SANTARPIA, Ralph, Peter, Edison, NJ 08820 (US); MELLO, Sarita, V., Somerset, NJ 08873 (US); ZAIDEL, Lynette, Cranford, NJ 07016 (US); CHOPRA, Suman, K., Monroe, NJ 08831 (US); WANG, Qin, Monmouth Junction, NJ 08852 (US); TAMBS, Gary, Edward, Belle Mead, NJ 08502 (US); BARNES, Virginia, Monsul, Ringoes, NJ 08551 (US); MORGAN, Andre, M., Robbinsville, NJ 08691 (US); KOHLI, Rajnish, Hillsborough, NJ 08844 (US); ROBINSON, Richard, Scott, Belle Mead, NJ 08502 (US); LEITE, Sergio, Kendall Park, NJ 08824 (US); SIMON, Eric, A., Somerset, NJ 08873 (US)
(74) Representative: Wibbelmann, Jobst
(86) International application number: PCT/US2008/058658
(87) International publication number: WO 2009/099451

(56) References cited:
- EP-A2- 0 569 666
- WO-A-00/78270
- WO-A-97/32565
- FR-A- 2 838 339
- GB-A- 2 354 441
- GB-A- 2 355 658
- US-A- 5 275 805
- US-A1- 2006 140 879
- US-A1- 2007 014 740
- DATABASE WPI Week 198527 Thomson Scientific, London, GB; AN 1985-162122 XP002505867 & JP 60 092208 A (KAO CORP) 23 May 1985 (1985-05-23)
- DATABASE WPI Week 200620 Thomson Scientific, London, GB; AN 2006-185073 XP002505868 & CN 1 569 866 A (UNIV. CHINA OCEANOGRAPHY) 26 January 2005 (2005-01-26)
- DATABASE WPI Week 200737 Thomson Scientific, London, GB; AN 2007-393500 XP002505869 & JP 2007 112766 A (POLA CHEM IND INC) 10 May 2007 (2007-05-10)
- DATABASE WPI Week 199312 Thomson Scientific, London, GB; AN 1993-096733 XP002530591 & JP 05 039213 A (LION CORP.) 19 February 1993 (1993-02-19)
- DATABASE WPI Week 200031 Thomson Scientific, London, GB; AN 2000-353463 XP002530592 & JP 2000 106845 A (KOGA) 18 April 2000 (2000-04-18)
- DATABASE WPI Week 198348 Thomson Scientific, London, GB; AN 1983-829923 XP002530593 & JP 58 180418 A (SANKIN KOGYO KK) 21 October 1983 (1983-10-21)
- DATABASE WPI Week 199651 Thomson Scientific, London, GB; AN 1996-514874 XP002530594 & JP 08 268853 A (LION CORP) 15 October 1996 (1996-10-15)
- DATABASE WPI Week 200624 Thomson Scientific, London, GB; AN 2006-222868 XP002530595 & CN 1 660 422 A (DEWEN BIOTECH DEV. CO. LTD.) 31 August 2005 (2005-08-31)
- DATABASE WPI Week 200423 Thomson Scientific, London, GB; AN 2004-242826 XP002530596 & JP 2003 342140 A (KAO CORP.) 3 December 2003 (2003-12-03)

## Description

### CROSS-REFERENCE TO RELATED APPLICATIONS

### FIELD OF THE INVENTION

This invention relates to novel salts of arginine and compositions comprising them.

### BACKGROUND OF THE INVENTION

Arginine and other basic amino acids have been proposed for use in oral care and are believed to have significant benefits in combating cavity formation and tooth sensitivity. Combining these basic amino acids with minerals having oral care benefits, e.g., fluoride and calcium, to form an oral care product having acceptable long term stability, however, has proven challenging. In particular, the basic amino acid may raise the pH and facilitate dissociation of calcium ions that can react with fluoride ions to form an insoluble precipitate. Moreover, the higher pH has the potential to cause irritation. At neutral pH or acidic pH, however, a system utilizing arginine bicarbonate (which the art teaches is preferred) may release carbon dioxide, leading to bloating and bursting of the containers. Moreover, it might be expected that lowering the pH to neutral or acidic conditions would reduce the efficacy of the formulation because the arginine may form an insoluble arginine-calcium complex that has a poorer affinity for the tooth surface, and moreover that lowering the pH would reduce any effect the formulation might have on buffering cariogenic lactic acid in the mouth. Partly because of these unaddressed formulation hurdles and partly because arginine has generally been viewed in the art as a potential alternative to fluoride rather than a co-active, there has been little motivation to make oral care products comprising both arginine and fluoride. Additional hurdles are potentially posed by addition of an antimicrobial agent. Commercially available arginine-based toothpaste, such as ProClude® and DenClude®, for example, contain arginine bicarbonate and calcium carbonate, but not fluoride nor any antimicrobial agent.

WO97/32565 describes anti-caries oral compositions containing calcium, arginine and a cariostatic anion.

At the same time, the value of antimicrobial agents, such as triclosan, in toothpaste has been recognized by many dentists. These agents however are challenging to deliver in effective amounts to the teeth and gums, and their solubility, delivery and retention on the teeth is formulation dependent. For example, triclosan (5-chloro-2-(2,4-dichlorophenoxy)phenol) is only slightly soluble in water.

Accordingly, there is a need for a stable oral care product providing arginine, as well as improved delivery of beneficial minerals such as fluoride and calcium, and of poorly soluble active agents.

### SUMMARY OF THE INVENTION

It has been surprisingly discovered that these problems can be addressed using functional salts of arginine.

The invention thus provides in one embodiment a salt of arginine and a polyphosphonic acid in solid form. Also described herein is a salt of arginine and one or more of the following cations
i. an acidic polymer e.g. a polycarboxylate polymer,
ii. the conjugate acid of an anionic surfactant e.g. lauroyl sulfuric acid,
iii. a polyphosphoric acid e.g. hexametaphosphoric acid or
iv. an acidic antimicrobial agent e.g. gallic acid.

In other embodiments, the invention provides oral and personal care products comprising the salts of the invention, and methods of making and using the salts and products.

The salt can be used in compositions to promote oral health and/or systemic health, including cardiovascular health, e.g., by reducing potential for systemic infection via the oral tissues.

### DETAILED DESCRIPTION

Herein described is Salt 1.0 a salt of arginine and an acidic polymer, e.g., a polymeric polycarboxylate, for example,
1.0.1. Salt 1.0 wherein the polymeric polycarboxylate is a 1:4 to 4:1 copolymer of maleic anhydride or acid with another polymerizable ethylenically unsaturated monomer.
1.0.2. Salt 1.0.1 wherein the polycarboxylate comprises units formed of maleic anhydride and
   methyl vinyl ether, e.g., depicted at pH 7 as follows: 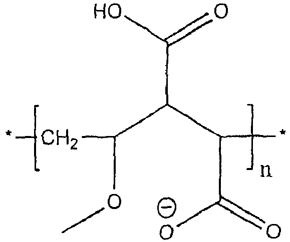 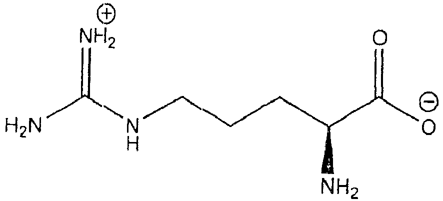
1.0.3. Salt 1.0.2 wherein the methyl vinyl ether/maleic anhydride has a molecular weight (M.W.) of about 30,000 to about 1,000,000.
1.0.4. Salt 1.0.3 wherein the methyl vinyl ether/maleic anhydride has a molecular weight (M.W.) of about 700,000.

Also described herein is a salt of arginine and the conjugate acid of an anionic surfactant salt (Salt 2.0). For example salts as follows:
2.0.1. Salt 2.0 wherein the conjugate acid is of an anionic surfactant salt is selected from
   a. water-soluble salts of higher fatty acid monoglyceride monosulfate (e.g., salt of the monosulfated monoglyceride of hydrogenated coconut oil fatty acids such as sodium N-methyl N-cocoyl taurate, sodium cocomo-glyceride sulfate),
   b. higher alkyl sulfates, e.g., sodium lauryl sulfate,
   c. higher alkyl-ether sulfates, e.g., of formula CH₃(CH₂)ₘCH₂(OCH₂CH₂)ₙOSO₃H, wherein m is 6-16, e.g., 10, n is 1-6, e.g., 2, 3 or 4, and X is Na or K (for example sodium laureth-2 sulfate (CH₃(CH₂)₁₀CH₂(OCH₂CH₂)₂OSO₃Na)),
   d. higher alkyl aryl sulfonates (such as sodium dodecyl benzene sulfonate (sodium lauryl benzene sulfonate)),
   e. higher alkyl sulfoacetates (such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate),
   f. and mixtures thereof;
   e.g., wherein by "higher alkyl" is meant C₆₋₃₀ alkyl, for example C₈₋₁₈.
2.0.2. Salt 2.0 or 2.0.1 which is a salt of arginine and lauroyl sulfuric acid.

The invention provides arginine polyphosphonate salts (Salt 3.0) in solid form. Also described herein are arginine polyphosphate salts, for example:
3.0.1. Salt 3.0 when a salt of arginine and polyvinyl phosphonic acid.
3.0.2. Salt 3.0 when a salt of arginine and a polyphosphoric acid.
3.0.3. Salt 3.0 when a salt of arginine and hexametaphosphoric acid.
3.0.4. Salt 3.0 when a salt of arginine and pyrophosphoric acid.
3.0.5. Salt 3.0 when a salt of arginine and a tripolyphosphate salt.

Also described herein are salts of arginine and an antibacterial acid (Salt 4.0). Also described herein (Salt 4.0.1), are salts of arginine and a benzoic acid optionally substituted with carboxy and/or one or more, e.g., 1, 2, or 3 hydroxys, e.g., benzoic acid, phthalic acid, salicylic acid or trihydroxybenzoic acid, for example, gallic acid.

The invention further provides an oral care composition comprising Salt 3.0. Also described herein are oral compositions comprising any of Salts 1.0 - 1.0.4; 2.0 - 2.0.2, 3.0.5 or 4.0 - 4.0.1, (Composition 1.1) e.g.,
1.1.1. Composition 1.1 further comprising an antibacterial agent. The antibacterial agent may be selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.1.2. Composition 1.1.1 wherein the antibacterial agent is triclosan.
1.1.3. Any of the preceding composition comprising triclosan and Zn²⁺ ion source, e.g., zinc citrate.
1.1.4. Any of the preceding compositions further comprising an anti-calculus agent.
1.1.5. Any of the preceding compositions further comprising an anti-calculus agent which is a polyphosphate, e.g., pyrophosphate, tripolyphosphate, or hexametaphosphate, e.g., in sodium salt form.
1.1.6. Any of the foregoing Compositions comprising a fluoride ion source.
1.1.7. Composition 1.1.6 wherein the fluoride ion source is a soluble fluoride salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof. 1.1.8. Any of the foregoing Compositions further comprising a surfactant.
1.1.9. Any of the compositions further comprising an agent that interferes with or prevents bacterial attachment, e.g., solbrol or chitosan.
1.1.10. Any of the foregoing Compositions in the form of a toothpaste optionally further comprising one or more of one or more of water, abrasives, surfactants, foaming agents, vitamins, polymers, enzymes, humectants, thickeners, antimicrobial agents, preservatives, flavorings, colorings and/or combinations thereof.

Also described herein is a method to improve oral health comprising applying an effective amount of Salt 3.0. Also described herein is a method comprising applying an effective amount of any of Salts 1.0 - 1.0.4; 2.0 - 2.0.2, 3.0.5 or 4.0 - 4.0.1, or any of Compositions 1.1 - 1.1.9 to the oral cavity of a subject in need thereof to
i. reduce or inhibit formation of dental caries,
ii. reduce, repair or inhibit early enamel lesions,
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial biofilm formation in the oral cavity,
x. raise and/or maintain plaque pH at levels of at least pH about 5.5 following sugar challenge,
xi. reduce plaque accumulation,
xii. treat, relieve or reduce dry mouth,
xiii. whiten teeth,
xiv. enhance systemic health, including cardiovascular health, e.g., by reducing potential for systemic infection via the oral tissues,
xv. reduce erosion of the teeth,
xvi. immunize the teeth against cariogenic bacteria, and/or
xvii. clean the teeth and oral cavity.
The invention further provides the use of any of a salt as claimed for any of the above-listed indications. Also described herein is the use of Salts 1.0 - 1.0.4; 2.0 - 2.0.2, 3.0 - 3.0.5 or 4.0 - 4.0.1, or any of Compositions 1.1 - 1.1.9, for any of the above-listed indications.

In various embodiments of the Compositions of the invention, the basic amino acid is present in an amount of about 0.5 wt. % to about 20 wt. % of the total composition weight, about 1 wt. % to about 10 wt. % of the total composition weight, for example about 1.5 wt. %, 3.75 wt. %, 5 wt. %, or 7.5 wt. % of the total composition weight.

### Fluoride Ion Source

The oral care compositions further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al., incorporated herein by reference.

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof.

In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A mouthwash, for example, would typically have about 100 to about 250 ppm fluoride. A toothpaste for general consumer use would typically have about 1000 to about 1500 ppm, with pediatric toothpaste having somewhat less. A dentifrice or coating for professional application could have as much as about 5,000 or even about 25,000 ppm fluoride.

Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt.

### Abrasives

The Compositions of the Invention may comprise a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate.

The compositions may include one or more additional abrasives, for example silica abrasives such as precipitated silicas having a mean particle size of up to about 20 microns, such as Zeodent 115^{®} marketed by J. M. Huber. Other useful abrasives also include sodium metaphosphate, potassium metaphosphate, aluminum silicate, calcined alumina, bentonite or other siliceous materials, or combinations thereof.

The silica abrasive polishing materials useful herein, as well as the other abrasives, generally have an average particle size of about 0.1 to about 30 microns, about 5 to about 15 microns. The silica abrasives can be from precipitated silica or silica gels, such as the silica xerogels described in U.S. Pat. No. 3,538,230, to Pader et al. and U.S. Pat. No. 3.862,307. to Digiulio, both incorporated herein by reference. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 115 and 119. These silica abrasives are described in U.S. Pat. No. 4,340,583. to Wason, incorporated herein by reference.

In certain embodiments, abrasive materials useful in the practice of the oral care compositions in accordance with the invention include silica gels and precipitated amorphous silica having an oil absorption value of about less than 100 cc/100 g silica or about 45 cc/100 g to about 70 cc/100 g silica. Oil absorption values are measured using the ASTM Rub-Out Method D281. In certain embodiments, the silicas are colloidal particles having an average particle size of about 3 microns to about 12 microns, and about 5 to about 10 microns.

In particular embodiments, the abrasive materials comprise a large fraction of very small particles, e.g., having a d50 less than about 5 microns, for example, small particle silica (SPS) having a d50 of 3-4 microns, for example Sorbosil AC43® (Ineos). Such small particles are particularly useful in formulations targeted at reducing hypersensitivity. The small particle component may be present in combination with a second larger particle abrasive. In certain embodiments, for example, the formulation comprises about 3 to about 8% SPS and about 25 to about 45% of a conventional abrasive.

Low oil absorption silica abrasives particularly useful in the practice of the invention are marketed under the trade designation Sylodent XWA^{®} by Davison Chemical Division of W.R. Grace & Co.. Baltimore. Md. 21203. Sylodent 650 XWA^{®}. a silica hydrogel composed of particles of colloidal silica having a water content of 29% by weight averaging about 7 to about 10 microns in diameter. and an oil absorption of less than about 70 cc/100 g of silica is an example of a low oil absorption silica abrasive useful in the practice of the present invention. The abrasive is present in the oral care composition of the present invention at a concentration of about 10 to 60% by weight, in other embodiment about 20 to 45% by weight, and in another embodiment about 30 to 50% by weight.

### Agents to Increase the Amount of Foaming

The oral care compositions of the invention also may include an agent to increase the amount of foam that is produced when the oral cavity is brushed.

Illustrative examples of agents that increase the amount of foam include, but are not limited to polyoxyethylene and certain polymers including, but not limited to, alginate polymers.

The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7.000.000. In one embodiment the molecular weight will be about 600,000 to about 2.000,000 and in another embodiment about 800,000 to about 1,000,000. Polyox^{®} is the trade name for the high molecular weight polyoxyethylene produced by Union Carbide.

The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e.. a single dose) is about 0.01 to about 0.9 % by weight, about 0.05 to about 0.5% by weight, and in another embodiment about 0.1 to about 0.2 % by weight.

### Surfactants

Another agent optionally included in the oral care composition of the invention is a surfactant or a mixture of compatible surfactants. Suitable surfactants arc those which are reasonably stable throughout a wide pH range, for example, anionic, cationic, nonionic or zwitterionic surfactants.

Suitable surfactants are described more fully, for example, in U.S. Pat. No. 3,959,458. to Agricola et al.; U.S. Pat. No. 3,937,807, to Haefele: and U.S. Pat. No. 4,051,234, to Gieske et al., which are incorporated herein by reference.

In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. Mixtures of anionic surfactants may also be utilized.

In another embodiment, cationic surfactants useful in the present invention can be broadly defined as derivatives of aliphatic quaternary ammonium compounds having one long alkyl chain containing about 8 to about 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride. and mixtures thereof.

Illustrative cationic surfactants are the quaternary ammonium fluorides described in U.S. Pat. No. 3,535,421, to Briner et al.. herein incorporated by reference. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides. long chain dialkyl sulfoxides and mixtures of such materials.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains about 8 to about 18 carbon atoms and one contains an anionic water-solubilizing group, e.g., carboxy, sulfonate, sulfate, phosphate or phosphonate. Illustrative examples of the surfactants suited for inclusion into the composition include, but are not limited to. sodium alkyl sulfate, sodium lauroyl sarcosinate, cocoamidopropyl betaine and polysorbate 20, and combinations thereof.

In a particular embodiment, the Composition of the Invention comprises an anionic surfactant, e.g.. sodium lauryl sulfate.

The surfactant or mixtures of compatible surfactants can be present in the compositions of the present invention in about 0.1 % to about 5.0%, in another embodiment about 0.3% to about 3.0% and in another embodiment about 0.5% to about 2.0% by weight of the total composition.

### Flavoring Agents

The oral care compositions of the invention may also include a flavoring agent. Flavoring agents which are used in the practice of the present invention include, but are not limited to. essential oils as well as various flavoring aldehydes, esters, alcohols, and similar materials. Examples of the essential oils include oils of spearmint, peppermint, wintergreen. sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of about 0.1 to about 5% by weight and about 0.5 to about 1.5% by weight. The dosage of flavoring agent in the individual oral care composition dosage (i.e.. a single dose) is about 0.001 to 0.05% by weight and in another embodiment about 0.005 to 0.015 % by weight.

### Chelating agents

The oral care compositions of the invention also may optionally include one or more chelating agents able to complex calcium found in the cell walls of the bacteria. Binding of this calcium weakens the bacterial cell wall and augments bacterial lysis.

Another group of agents suitable for use as chelating agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 1.0 wt. % pyrophosphate ions, about 1.5 wt. % to about 6 wt. %, about 3.5 wt. % to about 6 wt. % of such ions.

### Polymers

The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums. for example xanthan gum or carrageenan gum). Acidic polymers, for example polyacrylate gels. may be provided in the form of their free acids or partially or fully neutralized water soluble alkali metal (e.g., potassium and sodium) or ammonium salts.

Particularly when noncationic antibacterial agents or antibacterial agents, e.g., triclosan, are included in any of the dentifrice components, there is also preferably included from about 0.05 to about 5% of an agent which enhances the delivery and retention of the agents to, and retention thereof on oral surfaces. Such agents useful in the present invention are disclosed in U.S. Pat. Nos. 5,188,821 and 5,192,531; and include synthetic anionic polymeric polycarboxylates. such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 30,000 to about 800,000. These copolymers are available for example as Gantrez. e.g., AN 139 (M.W. 500,000), AN 119 (M.W. 250.000) and preferably S-97 Pharmaceutical Grade (M.W. 700,000) available from ISP Technologies. Inc., Bound Brook, N.J. 08805. The enhancing agents when present are present in amounts of about 0.05 to about 3% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic. 2-benzyl acrylic. 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1.000 to about 2.000,000, described in U.S. Pat. No. 4,842,847, Jun. 27, 1989 to Zahid, incorporated herein by reference.

Another useful class of polymeric agents includes polyamino acids, particularly those containing proportions of anionic surface-active amino acids such as aspartic acid, glutamic acid and phosphoserine, as disclosed in U.S. Pat. No. 4,866.161 Sikes et al., incorporated herein by reference.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Enzymes

The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420, U.S. Pat. No. 4,355,022; U.S. Pat. No. 4,154,815; U.S. Pat. No. 4,058,595; U.S. Pat. No. 3,991,177; and U.S. Pat. No. 3,696,191 all incorporated herein by reference. An enzyme of a mixture of several compatible enzymes in the current invention constitutes about 0.002% to about 2.0% in one embodiment or about 0.05% to about 1.5% in another embodiment or in yet another embodiment about 0.1% to about 0.5%.

### Water

Water may also be present in the oral compositions of the invention. Water, employed in the preparation of commercial oral compositions should be deionized and free of organic impurities. Water commonly makes up the balance of the compositions and includes about 10% to about 90%, about 20% to about 60% or about 10% to about 30% by weight of the oral compositions. This amount of water includes the free water which is added plus that amount which is introduced with other materials such as with sorbitol or any components of the invention.

### Humectants

Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70% in one embodiment or about 30% to about 65% in another embodiment by weight of the dentifrice composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5.004,597, to Majeti; U.S. Pat. No. 3,959,458 to Agricola et al. and U.S. Pat. No. 3,937,807. to Haefele, all being incorporated herein by reference.

### Methods of Manufacture

The compositions of the present invention can be made using methods which are common in the oral product area.

In one illustrative embodiment, the Salts are made by neutralizing arginine in a gel phase with the conjugate acid and mixing to form Premix 1. adjusting the pH to the desired level, and then combining with the other ingredients. Actives such as, for example, vitamins, CPC, fluoride, abrasives, and any other desired active ingredients are added to Premix 1 and mixed to form Premix 2. Where the final product is a toothpaste, a toothpaste base, for example, dicalcium phosphate or silica, is added to Premix 2 and mixed. The final slurry is formed into an oral care product.

### Composition Use

The present invention in its method aspect involves applying to the oral cavity a safe and effective amount of the compositions described herein.

The compositions and methods according to the invention are useful to a method to protect the teeth by facilitating repair and remineralization, in particular to reduce or inhibit formation of dental caries, reduce or inhibit demineralization and promote remineralization of the teeth, reduce hypersensitivity of the teeth, and reduce, repair or inhibit early enamel lesions, e.g., as detected by quantitative light-induced fluorescence (QLF) or electronic caries monitor (ECM).

Quantitative Light-induced Fluorescence is a visible light fluorescence that can detect early lesions and longitudinally monitor the progression or regression. Normal teeth fluoresce in visible light; demineralized teeth do not or do so only to a lesser degree. The area of demineralization can be quantified and its progress monitored. Blue laser light is used to make the teeth auto fluoresce. Areas that have lost mineral have lower fluorescence and appear darker in comparison to a sound tooth surface. Software is used to quantify the fluorescence from a white spot or the area/volume associated with the lesion. Generally, subjects with existing white spot lesions are recruited as panelists. The measurements are performed in vivo with real teeth. The lesion area/volume is measured at the beginning of the clinical. The reduction (improvement) in lesion area/volume is measured at the end of 6 months of product use. The data is often reported as a percent improvement versus baseline.

Electrical Caries Monitoring is a technique used to measure mineral content of the tooth based on electrical resistance. Electrical conductance measurement exploits the fact that the fluid-filled tubules exposed upon demineralization and erosion of the enamel conduct electricity. As a tooth loses mineral, it becomes less resistive to electrical current due to increased porosity. An increase in the conductance of the patient's teeth therefore may indicate demineralization. Generally, studies are conducted of root surfaces with an existing lesion. The measurements are performed in vivo with real teeth. Changes in electrical resistance before and after 6 month treatments are made. In addition, a classical caries score for root surfaces is made using a tactile probe. The hardness is classified on a three point scale: hard, leathery, or soft. In this type of study, typically the results are reported as electrical resistance (higher number is better) for the ECM measurements and an improvement in hardness of the lesion based on the tactile probe score.

The Compositions of the invention are thus useful in a method to reduce early lesions of the enamel (as measured by QLF or ECM) relative to a composition lacking effective amounts of fluorine and/or arginine.

The Compositions of the Invention are additionally useful in methods to reduce harmful bacteria in the oral cavity, for example methods to reduce or inhibit gingivitis, reduce levels of acid producing bacteria, to increase relative levels of arginolylic bacteria, inhibit microbial biofilm formation in the oral cavity, raise and/or maintain plaque pH at levels of at least pH about 5.5 following sugar challenge, reduce plaque accumulation, and/or clean the teeth and oral cavity.

Finally, by increasing the pH in the mouth and discouraging pathogenic bacteria, the Compositions of the Invention are useful to promote healing of sores or cuts in the mouth.

The compositions and methods according to the invention can be incorporated into oral compositions for the care of the mouth and teeth such as toothpastes, transparent pastes, gels, mouth rinses, sprays and chewing gum.

Enhancing oral health also provides benefits in systemic health, as the oral tissues can be gateways for systemic infections. Good oral health is associated with systemic health, including cardiovascular health. The compositions and methods of the invention provide particular benefits because basic amino acids, especially arginine, are sources of nitrogen which supply NO synthesis pathways and thus enhance microcirculation in the oral tissues. Providing a less acidic oral environment is also helpful in reducing gastric distress and creates an environment less favorable to Heliobacter, which is associated with gastric ulcers. Arginine in particular is required for high expression of specific immune cell receptors, for example T-cell receptors, so that arginine can enhance an effective immune response. The compositions and methods of the invention are thus useful to enhance systemic health, including cardiovascular health.

As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

## Claims

1. A salt of arginine and a polyphosphonic acid in solid form.

2. An oral care composition comprising a salt of arginine and a polyphosphonic acid, and a fluoride ion source.

3. An oral care composition according to claim 2 for use in a method comprising applying an effective amount of the salt to the oral cavity of a subject in need thereof to
i. reduce or inhibit formation of dental caries,
ii. reduce, repair or inhibit early enamel lesions,
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial biofilm formation in the oral cavity,
x. raise and/or maintain plaque pH at levels of at least pH about 5.5 following sugar challenge,
xi. reduce plaque accumulation,
xii. treat, relieve or reduce dry mouth,
xiii. whiten teeth,
xiv. enhance systemic health, including cardiovascular health,
xv. reduce erosion of the teeth,
xvi. immunize the teeth against cariogenic bacteria, and/or
xvii. clean the teeth and oral cavity.

## Patentansprüche

1. Salz von Arginin und einer Polyphosphonsäure in fester Form.

2. Mundpflegezusammensetzung, die ein Salz von Arginin und einer Polyphosphonsäure und eine Fluoridionenquelle umfasst.

3. Mundpflegezusammensetzung nach Anspruch 2 zur Verwendung in einem Verfahren, das das Anwenden einer wirksamen Menge des Salzes an die Mundhöhle eines Subjekts, das dieser Behandlung bedarf, umfasst, um
i. eine Bildung von Karies zu verringern oder zu verhindern,
ii. frühe Läsionen des Zahnschmelzes zu verringern, zu reparieren oder zu verhindern,
iii. eine Demineralisation zu verringern oder zu verhindern und eine Remineralisation der Zähne zu fördern,
iv. eine Überempfindlichkeit der Zähne zu verringern,
v. eine Zahnfleischentzündung zu verringern oder zu verhindern,
vi. eine Heilung von wunden Stellen oder Schnittstellen im Mund zu fördern,
vii. die Spiegel von Säure produzierenden Bakterien zu senken,
viii. die relativen Spiegel von arginolytischen Bakterien zu erhöhen,
ix. eine Bildung eines mikrobiellen Biofilms in der Mundhöhle zu verhindern,
x. den pH-Wert von Plaque auf einen Mindest-pH-Wert von etwa 5,5 nach einer Zuckerbelastung zu erhöhen und/oder auf diesem zu halten,
xi. eine Plaqueansammlung zu verringern,
xii. Mundtrockenheit zu behandeln, zu lindern oder zu verringern,
xiii. Zähne zu weißen,
xiv. die Allgemeingesundheit, einschließlich der kardiovaskulären Gesundheit, zu verbessern,
xv. eine Erosion der Zähne zu verringern,
xvi. die Zähne gegen kariogene Bakterien zu immunisieren, und/oder
xvii. die Zähne und die Mundhöhle zu reinigen.

## Revendications

1. Un sel d'arginine et un acide polyphosphonique sous forme solide.

2. Une composition pour soins buccaux comprenant un sel d'arginine et un acide polyphosphonique et une source d'ions fluorure.

3. Une composition pour soins buccaux selon la revendication 2 destinée à être utilisée dans un procédé comprenant l'application d'une quantité efficace du sel à la cavité buccale d'un sujet en ayant besoin pour
i. réduire ou inhiber la formation de caries dentaires,
ii. réduire, réparer ou inhiber les lésions précoces de l'émail,
iii. réduire ou inhiber la déminéralisation et promouvoir la reminéralisation des dents,
iv. réduire l'hypersensibilité des dents,
v. réduire ou éliminer la gingivite,
vi. promouvoir la cicatrisation des plaies ou des coupures dans la bouche,
vii. réduire les taux des bactéries productrices d'acide,
viii. augmenter les taux relatifs de bactéries arginolytiques,
ix. inhiber la formation d'un biofilm microbien dans la cavité buccale,
x. augmenter et/ou maintenir le pH de la plaque dentaire à un niveau de pH égal ou supérieur à environ 5,5 à la suite d'un défi au sucre,
xi. réduire l'accumulation de plaque dentaire,
xii. traiter, soulager ou réduire la bouche sèche,
xiii. blanchir les dents,
xiv. améliorer la santé systémique, y compris la santé cardiovasculaire,
xv. réduire l'érosion des dents,
xvi. immuniser les dents contre les bactéries cariogènes, et/ou
xvii. nettoyer les dents et la cavité buccale.
